# EUROPEAN PATENT APPLICATION

(11) **EP 4 641 454 A1**
(43) Date of publication of application: **29.10.2025**
(21) Application number: 23906905.7
(22) Date of filing: 14.12.2023
(51) Int. Cl.: G06N 20/00, A41H 1/02, G06T 7/00, G06T 7/62

(54) **INFORMATION PROCESSING DEVICE, INFORMATION PROCESSING METHOD, AND INFORMATION PROCESSING PROGRAM**

(30) Priority: 19.12.2022 JP 2022202396; 19.12.2022 JP 2022202397
(71) Applicant: ZOZO, Inc., Chiba-shi, Chiba, 263-0023 (JP)
(72) Inventor: YAMADA, Takayasu, Chiba-shi, Chiba 263-0023 (JP); ONO, Kengo, Chiba-shi, Chiba 263-0023 (JP); KOJIMA, Eri, Chiba-shi, Chiba 263-0023 (JP); BO, Li, Auckland 1023 (NZ)
(74) Representative: Hoffmann Eitle
(86) International application number: PCT/JP2023/044940
(87) International publication number: WO 2024/135541

(57) **Abstract**

To enable appropriate measurement of a body shape size of a user for each purpose. An information processing device according to the present application includes an acquisition unit and a learning unit. The acquisition unit acquires, from a three-dimensional model of the user, information indicating a range of a length measurement target and data of a length of a line segment of the measurement target, the data being of a length according to a purpose and used for a predetermined purpose. The learning unit causes learning of the estimation model that estimates the length of the line segment of the measurement target used for the purpose from the three-dimensional model such that the length of the line segment estimated from the three-dimensional model becomes an actual measurement value of the length of the line segment of the measurement target.

## Description

### Field

The present invention relates to an information processing device, an information processing method, and an information processing program.

### Background

A conventional technology of measuring a body shape size of a user is known. For example, there is known a technology of creating a three-dimensional model of a user using clothing for body size measurement and acquiring size information regarding the body shape of the user.

### Citation List

### Patent Literature

Patent Literature 1: WO 2019/189846 A
Patent Literature 2: JP 2020-95633 A

### Summary

### Technical Problem

However, in the conventional technology, it is not possible to appropriately measure the body shape size of the user for each purpose.

The present application has been made in view of the above, and an object thereof is to enable appropriate measurement of a body shape size of a user for each purpose.

### Solution to Problem

According to the present application, an information processing device includes an acquisition unit that acquires information indicating a range being a length measurement target from a three-dimensional model of a user and data of a length of a line segment of the measurement target, the data being of a length according to a purpose and used for a predetermined purpose, and a learning unit that causes learning of an estimation model that estimates a length of the line segment of the measurement target used for the purpose from the three-dimensional model such that the length of the line segment estimated from the three-dimensional model becomes an actual measurement value of the length of the line segment of the measurement target. Advantageous Effects of Invention

According to an aspect of the embodiment, it is possible to appropriately measure a body shape size of a user for each purpose.

### Brief Description of Drawings

FIG. 1 is a diagram illustrating a configuration example of an information processing system according to an embodiment.
FIG. 2 is a diagram illustrating an example of information processing according to the embodiment.
FIG. 3A is a diagram illustrating an example of display of a three-dimensional model according to the embodiment.
FIG. 3B is a diagram illustrating an example of designation of a range according to the embodiment.
FIG. 4 is a diagram illustrating a configuration example of a business operator terminal according to the embodiment.
FIG. 5 is a diagram illustrating a configuration example of a first user terminal according to the embodiment.
FIG. 6 is a diagram illustrating a configuration example of a second user terminal according to the embodiment.
FIG. 7 is a diagram illustrating a configuration example of an information processing device according to the embodiment.
FIG. 8 is a diagram illustrating an example of a model learning data storage unit according to the embodiment.
FIG. 9 is a diagram illustrating an example of a second user information storage unit according to the embodiment.
FIG. 10 is a flowchart illustrating an example of information processing according to the embodiment.
FIG. 11 is a flowchart illustrating an example of information processing according to the embodiment.
FIG. 12 is a hardware configuration diagram illustrating an example of a computer that implements functions of the information processing device. Description of Embodiments

Hereinafter, modes (hereinafter referred to as "embodiment") for implementing an information processing device, an information processing method, and an information processing program according to the present application will be described in detail with reference to the drawings. The information processing device, the information processing method, and the information processing program according to the present application are not limited by the embodiment. In the following embodiments, the same parts are denoted by the same reference numerals, and redundant description will be omitted.

### (Embodiment)

In tailor-made clothing or the like, a service using a three-dimensional model of a user is conceivable. For example, it is conceivable that, by creating the three-dimensional model of the user using clothing for body size measurement, the three-dimensional model is used for tailor-made clothing or the like. For example, such clothing for body size measurement has elasticity, and a predetermined mark, pattern, or the like is arranged at a predetermined position, and a method of generating a three-dimensional model of the user by analyzing captured images obtained by capturing the user wearing such clothing for body size measurement from a plurality of directions is considered. In the method using the clothing for body size measurement as described above, there is a case where the measurement value of the portion not covered with the clothing for body size measurement such as the head and the fingertip cannot be acquired. Therefore, a method of acquiring a measurement value of each part by direct input of data, a technology of estimating a body size from video data (for example, an image, a moving image, or the like), or the like is also considered. Hereinafter, the clothing for body size measurement according to the embodiment may cover each part of the body such as the head and the fingertip. The body size measurement method according to the embodiment may be a method of acquiring a measurement value of each part by a technology of estimating a body size from directly input data or video data. In addition, the body size measurement method according to the embodiment may be a measurement technology that can measure the size of the foot by imaging the periphery of the foot (for example, ZOZOMAT), a measurement technology that can measure the size of the finger by imaging the periphery of the hand (for example, ZOZOMAT for Hands), a measurement technology that can measure the size of the breast by photographing the periphery of the breast (for example, ZOZOBRA), or the like.

Here, in a case where the three-dimensional model is generated from the captured image of the user wearing such clothing for body size measurement, it is necessary to measure the positional relationship (for example, in which direction and how much it is deviated) for all the points of the user's body in order to accurately match the positional relationship of each part of the three-dimensional model with the user. However, it is practically impossible to measure the positional relationship for all the points of the user's body in this manner.

Therefore, a method of measuring only a positional relationship of a predetermined mark among marks given to the clothing for body size measurement, that is, a positional relationship of a finite number of points, and generating a three-dimensional model based on a measurement result is considered. When the three-dimensional model is generated by such a method, since the shape of the entire three-dimensional model is estimated on the basis of the shape of a general human, an error occurs between the position of a point other than the point where the positional relationship is actually measured and the actual position. If complementation is not performed as this case, data of only the measured positions is obtained, the three-dimensional model becomes not smooth, and the utility value of the three-dimensional model decreases.

In a case where such a three-dimensional model is used, an accurate size cannot be specified depending on a measurement place, and a discrepancy occurs when the three-dimensional model is used for tailor-made or the like. In particular, in the case of estimating the shape of the entire three-dimensional model, estimation based on the shape of a general human is performed. Therefore, for example, in a case where a part of the body is changed due to various patients such as hallux valgus, fractures, or labor, the difference between the actual shape of the body of the user and the three-dimensional model further increases. Therefore, in the present application, a model for correcting the size is generated. In particular, such a model is generated for each business operator and for each group of users who use the business operator.

### [1. Configuration of information processing system]

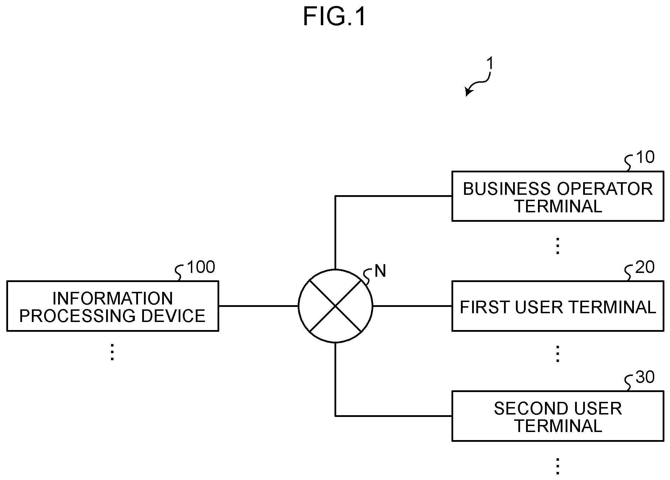

An information processing system 1 illustrated in FIG. 1 will be described. As illustrated in FIG. 1, the information processing system 1 includes a business operator terminal (terminal device)10, a first user terminal 20, a second user terminal 30, and an information processing device 100. The business operator terminal 10, the first user terminal 20, the second user terminal 30, and the information processing device 100 are communicably connected in a wired or wireless manner via a predetermined communication network (network N). FIG. 1 is a diagram illustrating a configuration example of the information processing system 1 according to the embodiment.

The business operator terminal 10 is an information processing device used by a business (vendor) that has a desire to appropriately grasp the body shape size of the user. The business terminal who uses the business operator terminal 10 is, for example, a business operator who has applied for a service provided by the information processing device 100. For example, the business operator who uses the business operator terminal 10 is a business operator who sells clothes, old clothes, and the like in a predetermined electronic shopping street. The business operator terminal 10 may be any device as long as the processing in the embodiment can be achieved. The business operator terminal 10 may be a device such as a smartphone, a tablet terminal, a notebook PC, a desktop PC, a mobile phone, or a PDA.

The first user terminal 20 is an information processing device used by a user who provides a length of a line segment of the measurement target estimated from a three-dimensional model and an actual measurement value of the length of the line segment of the measurement target for learning an estimation model for estimating a body shape size of the user. The user who uses the first user terminal 20 is, for example, a cooperator for data collection having a cooperative relationship with the business operator. The first user terminal 20 may be any device as long as the processing in the embodiment can be achieved. The first user terminal 20 may be a device such as a smartphone, a tablet terminal, a notebook PC, a desktop PC, a mobile phone, or a PDA.

The second user terminal 30 is an information processing device used by a user who uses a service provided by the information processing device 100. The user who uses the second user terminal 30 is, for example, an end user. The second user terminal 30 may be any device as long as the processing in the embodiment can be achieved. The second user terminal 30 may be a device such as a smartphone, a tablet terminal, a notebook PC, a desktop PC, a mobile phone, or a PDA.

The information processing device 100 is an information processing device intended to enable appropriate measurement of the body shape size of the user for each business operator, and may be any device as long as the processing in the embodiment can be achieved. The information processing device 100 is achieved by, for example, a server device, a cloud system, or the like. For example, the information processing device 100 issues an account in response to a service application from the business operator. For example, the information processing device 100 provides various services to the end user.

### [2. Example of information processing]

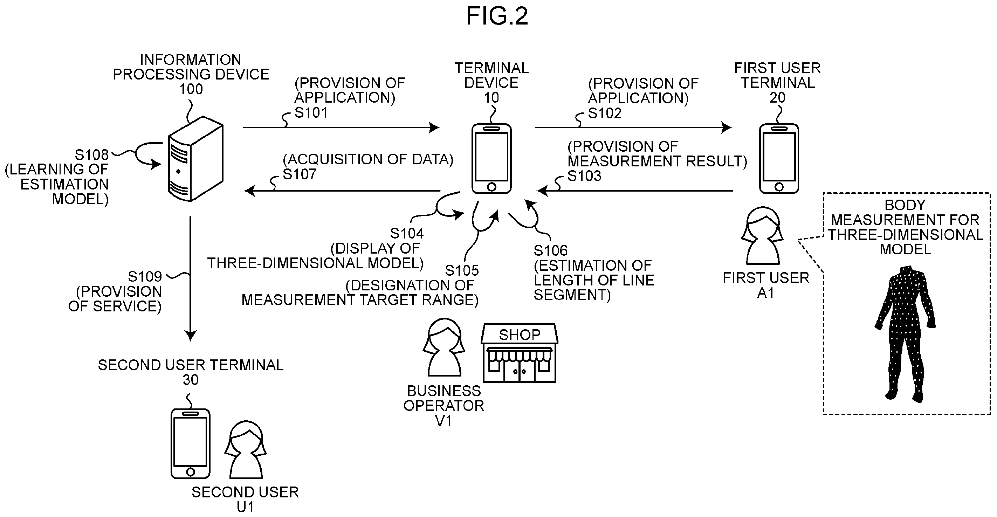

FIG. 2 is a diagram illustrating an example of information processing of the information processing system 1 according to the embodiment. In the following embodiment, the three-dimensional model is a model in which the entire size is estimated by measuring a part of the size. For example, the three-dimensional model according to the embodiment is a three-dimensional model in which the entire size of the user is estimated on the basis of data acquired from a clothing for body size measurement that can be worn by the user to measure the size of a specific portion. The three-dimensional model according to the embodiment may be a three-dimensional model of the first user or a three-dimensional model of the second user. A business operator V1 is a business operator that has applied for the service according to the embodiment. A first user A1 is a user (cooperator) having a cooperative relationship of data collection with the business operator V1. A second user U1 is a user (end user) who uses the service provided by the information processing device 100. Hereinafter, the first user and the second user may be collectively referred to as "users" as appropriate. The range being the measurement target according to the embodiment may be any part of the body of the user as long as it is a range that can be designated from the three-dimensional model, and is not particularly limited. Therefore, any portion may be set in the range being the measurement target for each business operator. The range being the measurement target according to the embodiment may be a position, and hereinafter, the range being the measurement target may be appropriately read as a "position being the measurement target".

When accepting the application for the service from the business operator V1, the information processing device 100 issues an account for the business operator V1 and provides application information for service use to the business operator V1 (Step S101).

The business operator V1 provides an application based on the application information provided from the information processing device 100 to the first user A1 who is a cooperator for data collection (Step S102).

The first user A1 measures the body size of the first user A1, and provides the measurement result to the business operator V1 via the application based on the application information provided from the business operator terminal 10 (Step S103). For example, the first user terminal 20 transmits the measurement result of the body shape size of the first user A1 to the business operator terminal 10.

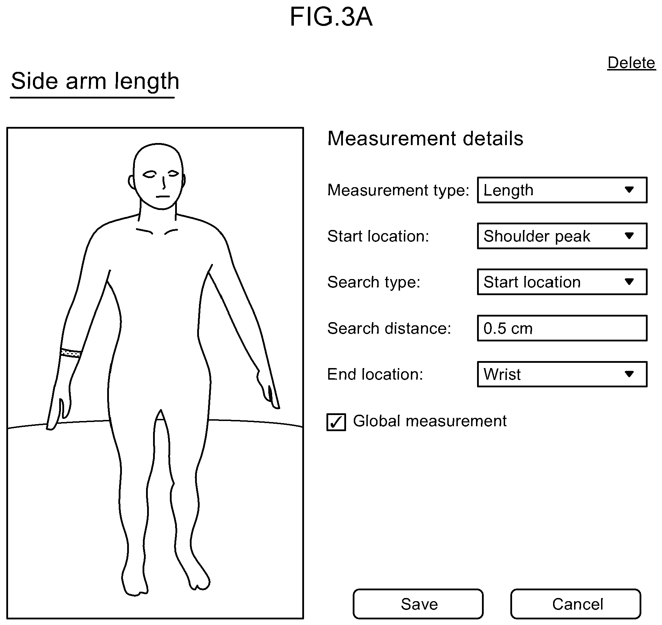

When acquiring the measurement result of the body shape size of the first user A1, the business operator terminal 10 displays the three-dimensional model of the body shape of the first user A1 on the basis of the acquired measurement result via the application provided from the information processing device 100 (Step S104). FIG. 3A is a diagram illustrating an example of display of a three-dimensional model according to the embodiment.

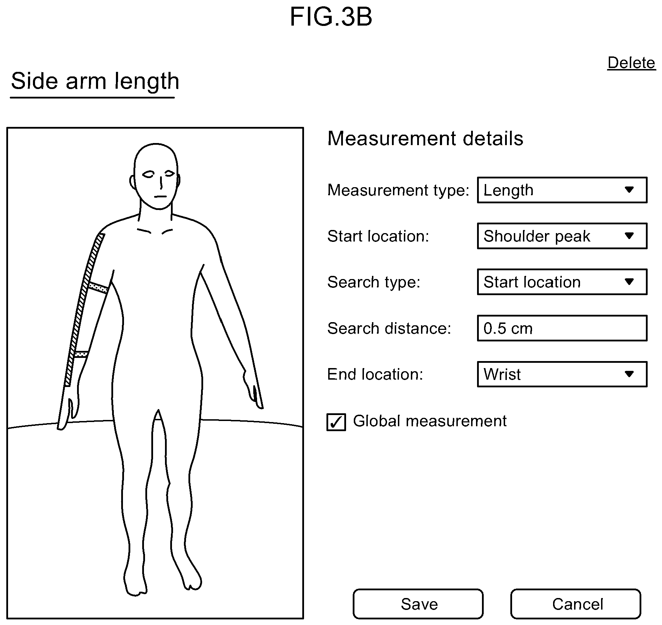

When the business operator V1 designates a range to be a measurement target of the length from the three-dimensional model displayed on the business operator terminal 10 via the application provided from the information processing device 100, the business operator terminal 10 receives designation of the range by the operator V1 (Step S105). FIG. 3B is a diagram illustrating an example of designation of a range according to the embodiment. The business operator V1 may designate the range from the left image or may designate the range from the right input field. In FIG. 3B, since the range from the wrist to the shoulder of the right arm of the three-dimensional model is selected, this range is designated.

The business operator terminal 10 estimates the length of a line segment in a designated range from the three-dimensional model via the application provided from the information processing device 100 (Step S106). The business operator V1 measures (for example, measurement is performed with a hand measure or the like) the actual measurement value of the length of the line segment of the measurement target of the first user A1, and inputs the measurement result to the business operator terminal 10, so that the business operator terminal 10 receives the actual measurement value of the length of the line segment of the measurement target of the first user A1.

The information processing device 100 acquires information indicating the range being the measurement target designated by the business operator V1 and data (an estimated value of a length of a line segment estimated from a three-dimensional model, and actual measurement value of the length of the line segment of the measurement target) of the length of the line segment of the measurement target of the first user A1 (Step S107). Then, the information processing device 100 causes the estimation model that estimates the length of the line segment of the measurement target from the three-dimensional model to learn such that the length of the line segment estimated from the three-dimensional model becomes an actual measurement value of the length of the line segment of the measurement target (Step S108). The information processing device 100 may learn the estimation model by acquiring the information indicating the range being the measurement target designated by the business operator V1 and the data of the lengths of the line segments of the measurement target of a plurality of first users. For example, it is sufficient that, in a case where the range being the measurement target designated by the business operator V1 and the measured value of the length estimated from the three-dimensional model are input, the information processing device 100 learns the estimation model so as to output the actual measurement value of the length of the line segment of the measurement target. That is, the information processing device 100 may cause learning of the estimation model by using, as training data, the information indicating the range being the measurement target designated by the business operator V1 and the data of the lengths of the line segments of the measurement target of a plurality of first users. The information processing device 100 may cause learning of the estimation model for each business operator or may cause learning of the estimation model for each purpose. Even in the same business operator, for example, the measurement degree may be different between the case of measuring the length of the waist portion in the case of tailoring the jacket of the suit and the case of measuring the length of the waist portion in the case of tailoring the pants of the suit. For example, in a case where the measurement is performed in a tighter state in the case of the pants than in the case of the jacket, it is assumed that the measurement result of the length of the waist portion in the case of the pants tends to be thinner than the measurement result of the length of the waist portion in the case of the jacket. The information processing device 100 may cause learning of an estimation model that estimates the length of the line segment of the measurement target used for the purpose from the three-dimensional model by acquiring data of the length of the line segment of the measurement target of the first user, the data having a length according to the purpose used for the predetermined purpose.

For example, in a case where the information processing device 100 proposes, to the second user U1, a size that matches the second user U1 among the sizes of the products sold by the business operator V1, the information processing device 100 provides the second user U1 with a service for estimating the length of the line segment of the measurement target from the three-dimensional model using the estimation model for estimating the length of the line segment of the measurement target designated by the business operator V1 among the estimation models learned for each business (Step S109). For example, once the second user U1 measures the body shape size of the second user U1, the information processing device 100 stores the measurement information to provide a service that can be used by the service of each business operator.

### [3. Configuration of business operator terminal]

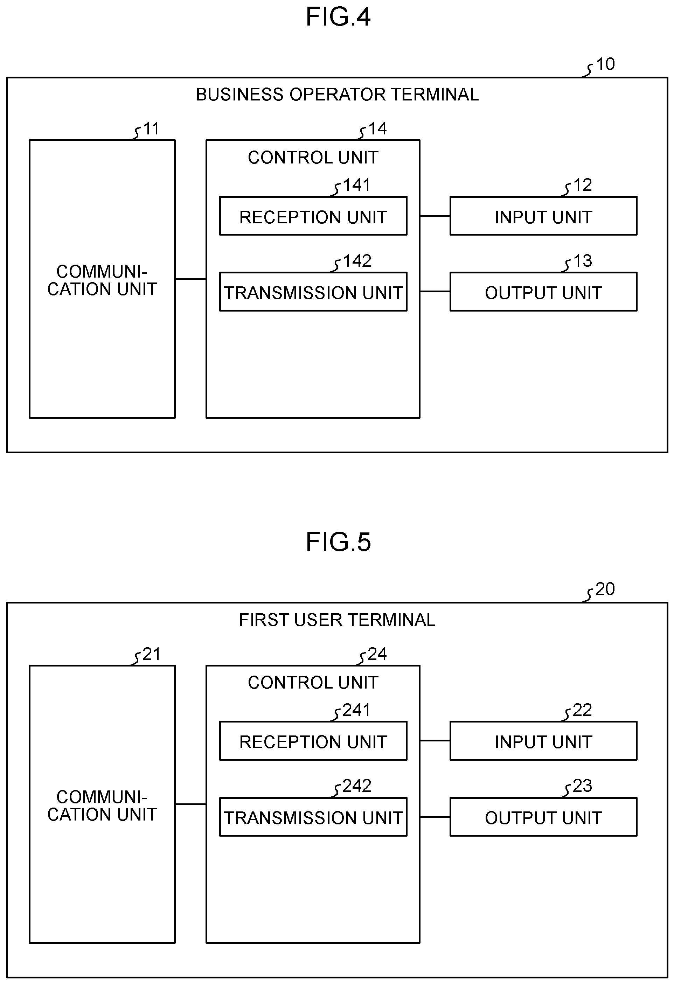

Next, a configuration of the business operator terminal 10 according to the embodiment will be described with reference to FIG. 4. FIG. 4 is a diagram illustrating a configuration example of the business operator terminal 10 according to the embodiment. As illustrated in FIG. 4, the business operator terminal 10 includes a communication unit 11, an input unit 12, an output unit 13, and a control unit 14.

### (Communication unit 11)

The communication unit 11 is achieved by, for example, a network interface card (NIC) or the like. Then, the communication unit 11 is connected to a predetermined network N in a wired or wireless manner, and transmits and acquires information to and from the information processing device 100 and the like via the predetermined network N.

### (Input unit 12)

The input unit 12 receives various operations from a business operator. In FIG. 2, the input unit 12 receives various operations from the business operator V1. For example, the input unit 12 may receive various operations from the business operator via a display surface by a touch panel function. The input unit 12 may receive various operations from a button provided on the business operator terminal 10 or a keyboard or a mouse connected to the business operator terminal 10.

### (Output unit 13)

The output unit 13 is a display screen of a tablet terminal or the like achieved by, for example, a liquid crystal display, an organic electro-luminescence (EL) display, or the like, and is a display device for displaying various types of information. For example, the output unit 13 displays information transmitted from the information processing device 100.

### (Control unit 14)

The control unit 14 is, for example, a controller, and is achieved by executing various programs stored in a storage device inside the business operator terminal 10 using a random access memory (RAM) as a work area by a central processing unit (CPU), a micro processing unit (MPU), or the like. For example, the various programs include programs of applications installed in the business operator terminal 10. For example, the various programs include an application program for executing an application based on application information transmitted from the information processing device 100. The control unit 14 is achieved by, for example, an integrated circuit such as an application specific integrated circuit (ASIC) or a field programmable gate array (FPGA).

As illustrated in FIG. 4, the control unit 14 includes a reception unit 141 and a transmission unit 142, and achieves or executes an action of information processing described below.

### (Reception Unit 141)

The reception unit 141 receives various types of information from another information processing device such as the information processing device 100. For example, the reception unit 141 receives application information transmitted from the information processing device 100. In addition, for example, the reception unit 141 receives data transmitted from the first user terminal 20 of the first user having a data collection cooperation relationship with the business operator.

### (Transmission unit 142)

The transmission unit 142 transmits various types of information to another information processing device such as the information processing device 100. For example, the transmission unit 142 transmits application information for a service received from the business operator to the information processing device 100. In addition, for example, the transmission unit 142 transmits, to the first user terminal 20, information that is included in the application information transmitted from the information processing device 100 and enables data collection.

### [4. Configuration of first user terminal]

Next, a configuration of the first user terminal 20 according to the embodiment will be described with reference to FIG. 5. FIG. 5 is a diagram illustrating a configuration example of the first user terminal 20 according to the embodiment. As illustrated in FIG. 5, the first user terminal 20 includes a communication unit 21, an input unit 22, an output unit 23, and a control unit 24.

### (Communication unit 21)

The communication unit 21 is achieved by, for example, an NIC or the like. Then, the communication unit 21 is connected to a predetermined network N in a wired or wireless manner, and transmits and acquires information to and from the business operator terminal 10 and the like via the predetermined network N.

### (Input unit 22)

The input unit 22 receives various operations from a first user. In FIG. 2, the input unit 22 receives various operations from the first user A1. For example, the input unit 22 may receive various operations from the first user via a display surface by a touch panel function. The input unit 22 may receive various operations from a button provided on the first user terminal 20 or a keyboard or a mouse connected to the first user terminal 20.

### (Output unit 23)

The output unit 23 is a display screen of a tablet terminal or the like achieved by, for example, a liquid crystal display, an EL display, or the like, and is a display device for displaying various types of information. For example, the output unit 23 displays information transmitted from the business operator terminal 10.

### (Control unit 24)

The control unit 24 is, for example, a controller, and is achieved by the CPU, the MPU, or the like executing various programs stored in the storage device inside the first user terminal 20 using the RAM as a work area. For example, the various programs include programs of applications installed in the first user terminal 20. For example, the various programs include an application program for executing an application based on application information transmitted from the business operator terminal 10. The control unit 24 is achieved by, for example, an integrated circuit such as an ASIC or an FPGA.

As illustrated in FIG. 5, the control unit 24 includes a reception unit 241 and a transmission unit 242, and achieves or executes an action of information processing described below.

### (Reception unit 241)

The reception unit 241 receives various types of information from another information processing device such as the business operator terminal 10. For example, the reception unit 241 receives application information transmitted from the business operator terminal 10 for data collection.

### (Transmission unit 242)

The transmission unit 242 transmits various types of information to another information processing device such as the business operator terminal 10. For example, the transmission unit 242 transmits data for data collection to the business operator terminal 10. For example, the transmission unit 242 transmits, to the business operator terminal 10, data for learning an estimation model for estimating a length of a line segment in a measurement target range designated by a specific business operator.

### [5. Configuration of second user terminal]

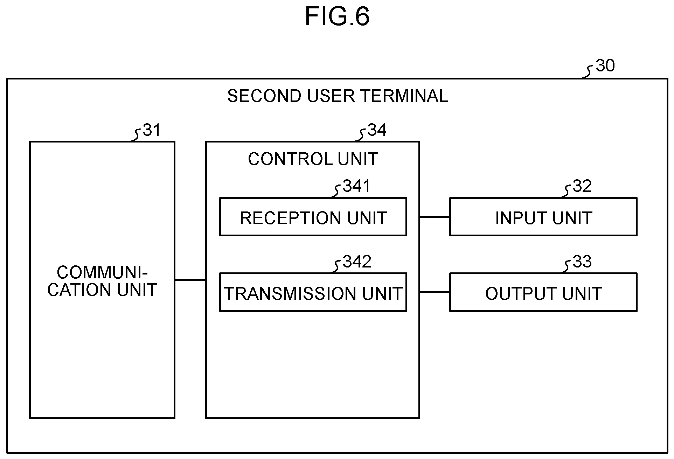

Next, a configuration of the second user terminal 30 according to the embodiment will be described with reference to FIG. 6. FIG. 6 is a diagram illustrating a configuration example of the second user terminal 30 according to the embodiment. As illustrated in FIG. 6, the second user terminal 30 includes a communication unit 31, an input unit 32, an output unit 33, and a control unit 34.

### (Communication unit 31)

The communication unit 31 is achieved by, for example, an NIC or the like. Then, the communication unit 31 is connected to a predetermined network N in a wired or wireless manner, and transmits and acquires information to and from the information processing device 100 and the like via the predetermined network N.

### (Input unit 32)

The input unit 32 receives various operations from a second user. In FIG. 2, the input unit 32 receives various operations from the second user U1. For example, the input unit 32 may receive various operations from the second user via a display surface by a touch panel function. The input unit 32 may receive various operations from a button provided on the second user terminal 30 or a keyboard or a mouse connected to the second user terminal 30.

### (Output unit 33)

The output unit 33 is a display screen of a tablet terminal or the like achieved by, for example, a liquid crystal display, an EL display, or the like, and is a display device for displaying various types of information. For example, the output unit 33 displays information transmitted from the information processing device 100.

### (Control unit 34)

The control unit 34 is, for example, a controller, and is achieved by the CPU, the MPU, or the like executing various programs stored in the storage device inside the second user terminal 30 using the RAM as a work area. For example, the various programs include programs of applications installed in the second user terminal 30. For example, the various programs include an application program for executing an application for using a service provided by the information processing device 100. The control unit 34 is achieved by, for example, an integrated circuit such as an ASIC or an FPGA.

As illustrated in FIG. 6, the control unit 34 includes a reception unit 341 and a transmission unit 342, and achieves or executes an action of information processing described below.

### (Reception unit 341)

The reception unit 341 receives various types of information from another information processing device such as the information processing device 100. For example, the reception unit 341 receives information for using a service provided by the information processing device 100.

### (Transmission unit 342)

The transmission unit 342 transmits various types of information to another information processing device such as the information processing device 100. For example, the transmission unit 342 transmits feedback for the service provided by the information processing device 100. The transmission unit 342 may transmit data for learning an estimation model for estimating the length of a line segment in a measurement target range designated by a specific business operator.

### [6. Configuration of information processing device]

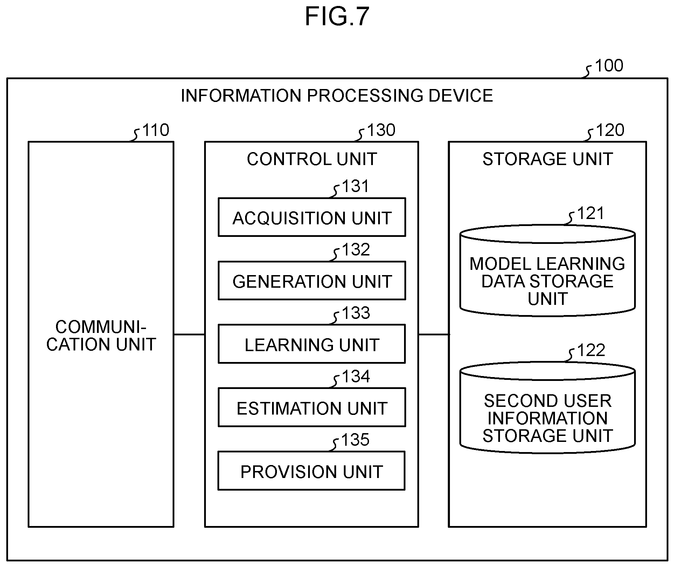

Next, a configuration of the information processing device 100 according to the embodiment will be described with reference to FIG. 7. FIG. 7 is a diagram illustrating a configuration example of the information processing device 100 according to the embodiment. As illustrated in FIG. 7, the information processing device 100 includes a communication unit 110, a storage unit 120, and a control unit 130. The information processing device 100 may include an input unit (for example, a keyboard, a mouse, or the like) that receives various operations from an administrator of the information processing device 100, and a display unit (for example, a liquid crystal display or the like) that displays various types of information.

### (Communication unit 110)

The communication unit 110 is achieved by, for example, an NIC or the like. Then, the communication unit 110 is connected to a network N in a wired or wireless manner, and transmits and acquires information to and from the business operator terminal 10 and the like via the network N.

### (Storage unit 120)

The storage unit 120 is achieved by, for example, a semiconductor memory element such as a RAM or a flash memory, or a storage device such as a hard disk or an optical disk. As illustrated in FIG. 7, the storage unit 120 includes a model learning data storage unit 121 and a second user information storage unit 122.

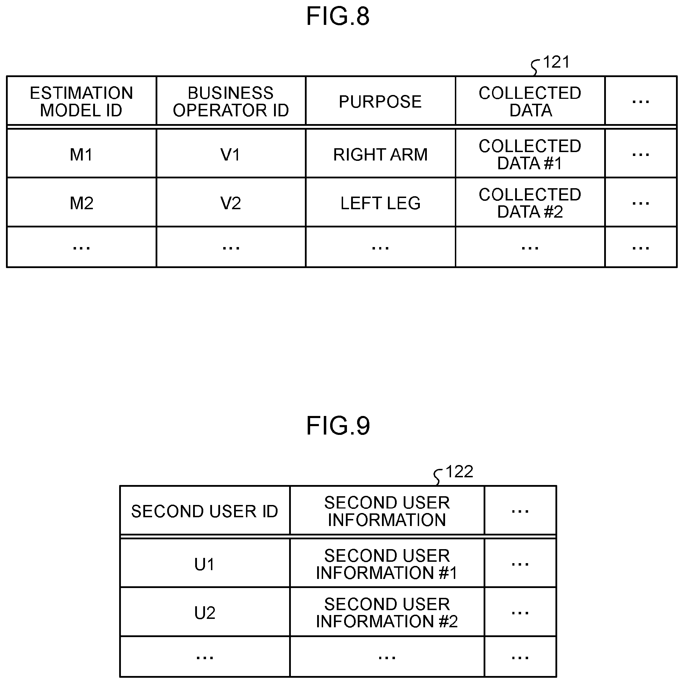

The model learning data storage unit 121 stores training data for learning an estimation model for estimating the length of the line segment of the measurement target. FIG. 8 illustrates an example of the model learning data storage unit 121 according to the embodiment. As illustrated in FIG. 8, the model learning data storage unit 121 includes items such as "estimation model ID", "business operator ID", "purpose", and "collected data".

The "estimation model ID" indicates identification information for identifying the estimation model. The "business operator ID" indicates identification information for identifying the business operator. The "purpose" indicates a predetermined purpose according to the embodiment. For example, the "purpose" indicates a measurement target range designated by the business operator. The "collected data" indicates data of a cooperator having a cooperative relationship with the business operator. For example, the "collected data" includes data of the length of the line segment of the measurement target estimated from the three-dimensional model and data of an actual measurement value of the length of the line segment of the measurement target. In the example illustrated in FIG. 8, an example in which conceptual information such as "collected data #1" and "collected data #2" is stored in "collected data" has been described, but actually, a numerical value or the like indicating a length of a line segment is stored.

The second user information storage unit 122 stores information on the second user. For example, the second user information storage unit 122 stores information on a three-dimensional model of the second user. Here, FIG. 9 illustrates an example of the second user information storage unit 122 according to the embodiment. As illustrated in FIG. 9, the second user information storage unit 122 includes items such as "second user ID" or "second user information".

The "second user ID" indicates identification information for identifying the second user. The "second user information" indicates second user information. For example, the "second user information" includes information on the three-dimensional model of the second user. In the example illustrated in FIG. 9, conceptual information such as "second user information #1" and "second user information #2" is stored in the "second user information". However, actually, a numerical value or the like indicating a length of a line segment of each part of the second user is stored.

### (Control unit 130)

The control unit 130 is a controller, and is implemented by, for example, a CPU, an MPU, or the like executing various programs stored in a storage device inside the information processing device 100 using a RAM as a work area. The control unit 130 is a controller and achieved by, for example, an integrated circuit such as an ASIC or an FPGA.

As illustrated in FIG. 7, the control unit 130 includes an acquisition unit 131, a generation unit 132, a learning unit 133, an estimation unit 134, and a provision unit 135, and achieves or executes an action of information processing described below. The internal configuration of the control unit 130 is not limited to the configuration illustrated in FIG. 7, and may be another configuration as long as information processing to be described later is performed.

### (Acquisition unit 131)

The acquisition unit 131 acquires various types of information from an external information processing device. The acquisition unit 131 acquires various types of information from another information processing device such as the business operator terminal 10.

The acquisition unit 131 acquires various types of information from the storage unit 120. The acquisition unit 131 stores the acquired various types of information in the storage unit 120.

The acquisition unit 131 acquires, from the three-dimensional model of the user, information indicating a range being the measurement target of a length designated by the business operator. The acquisition unit 131 acquires data of the length of the line segment of the measurement target. For example, the acquisition unit 131 acquires data of the length of the line segment of the measurement target estimated from the three-dimensional model. For example, the acquisition unit 131 acquires data of an actual measurement value of the length of the line segment of the measurement target.

### (Generation unit 132)

The generation unit 132 uses the three-dimensional model of the user as an input, and generates an estimation model that estimates the length of the line segment of the measurement target designated for each business operator. For example, the generation unit 132 generates an estimation model for each business operator on the basis of training data collected by collecting training data for each business operator. The generation unit 132 may generate an estimation model that estimates the length of the line segment of the measurement target for each purpose.

### (Learning unit 133)

The learning unit 133 causes learning of the estimation model generated by the generation unit 132 such that the length of the line segment estimated from the three-dimensional model of the first user becomes an actual measurement value of the length of the line segment of the measurement target of the first user. The learning unit 133 may cause learning of the estimation model generated by the generation unit 132 such that the length of the line segment estimated from the three-dimensional model of the second user becomes an actual measurement value of the length of the line segment of the measurement target of the second user. The learning unit 133 may cause learning of the estimation model every time the training data increases, or may cause learning of the estimation model for every predetermined period or every time the number of pieces of the training data reaches a predetermined threshold. As described above, the learning unit 133 may repeatedly perform learning to improve the accuracy of the estimation model. The number of pieces of training data may be settable for each business operator, and the business operator may freely make a change to increase the number of training data if it is necessary to increase the accuracy. The learning unit 133 may learn the estimation model including physical features such as hometown and race, external features such as a measurement time zone, and the like. For example, in the case of an external feature, since the morning is more likely to cause swelling than the night, the learning unit 133 may separately generate an estimation model for the morning and an estimation model for the night and separately perform learning. For example, in the case of a physical feature, since the legs of an athlete are thicker than the legs of a person who is not an athlete, the learning unit 133 may separately generate an estimation model for an athlete and an estimation model for a person who is not an athlete and separately perform learning.

### (Estimation unit 134)

The estimation unit 134 estimates the length of the line segment in the measurement target range designated by the specific business operator from the three-dimensional model of the second user using the estimation model learned by the learning unit 133. For example, the estimation unit 134 estimates the length of the line segment in the measurement target range designated by the business operator according to the service selected by the second user.

### (Provision unit 135)

The provision unit 135 transmits various types of information to the business operator terminal 10, the first user terminal 20, and the second user terminal 30. For example, the provision unit 135 provides the second user with information on the length of the line segment estimated by the estimation unit 134.

### [7. Flow of information processing]

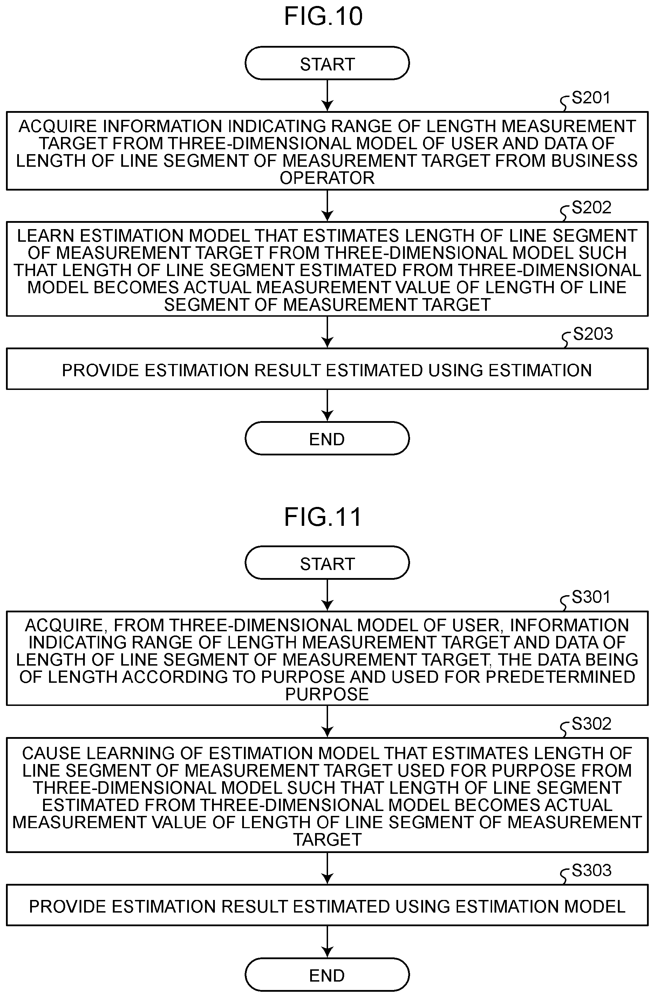

Next, a procedure of information processing by the information processing system 1 according to the embodiment will be described with reference to FIGS. 10 and 11. FIGS. 10 and 11 are flowcharts illustrating a procedure of information processing by the information processing system 1 according to the embodiment.

As illustrated in FIG. 10, the information processing device 100 acquires information indicating a range of a length measurement target from the three-dimensional model of the user and data of the length of a line segment of the measurement target from the business operator (Step S201).

The information processing device 100 causes learning of the estimation model that estimates the length of the line segment of the measurement target from the three-dimensional model such that the length of the line segment estimated from the three-dimensional model becomes an actual measurement value of the length of the line segment of the measurement target (Step S202).

The information processing device 100 provides an estimation result estimated using the estimation model (Step S203).

As illustrated in FIG. 11, the information processing device 100 acquires, from the three-dimensional model of the user, information indicating a range of a length measurement target and data of a length of a line segment of the measurement target, the data having a length according to a purpose used for a predetermined purpose (Step S301).

The information processing device 100 causes learning of the estimation model that estimates the length of the line segment of the measurement target used for the purpose from the three-dimensional model such that the length of the line segment estimated from the three-dimensional model becomes an actual measurement value of the length of the line segment of the measurement target (Step S302).

The information processing device 100 provides an estimation result estimated using the estimation model (Step S303).

### [8. Application example]

The above embodiment is expected to be particularly applied, for example, in a case where line segment information of each part of the body changes due to disease or the like. For example, the present invention may be applied to estimation of the length of the line segment of the measurement target of a patient (for example, a patient with a condition in which lymph fluid accumulates under the skin and becomes swollen) having a symptom that a specific site is swollen due to disease or the like. By application to the estimation of the length of the line segment of the measurement target of the user in which only the line segment information of the specific part has changed due to disease or the like, it is possible to appropriately estimate the length of the line segment of the measurement target of the user. Since only such a specific portion can estimate the length of the line segment of the measurement target of the user having swelling unlike usual, the embodiment can also be applied to the production of a clothing or the like for such a user. Such an application example is an example and is not particularly limited.

### [9. Effect (1)]

As described above, the information processing device 100 according to the embodiment includes the acquisition unit 131 and the learning unit 133. The acquisition unit 131 acquires, from the three-dimensional model of the user, information indicating a range of a length measurement target and data of a length of a line segment of the measurement target, the data having a length according to a purpose used for a predetermined purpose. The learning unit 133 causes learning of the estimation model that estimates the length of the line segment of the measurement target used for the purpose from the three-dimensional model such that the length of the line segment estimated from the three-dimensional model becomes an actual measurement value of the length of the line segment of the measurement target.

As a result, the information processing device 100 according to the embodiment acquires the information indicating the range being the measurement target and the data of the length of the line segment of the measurement target, which is the data of the length according to the purpose used for a predetermined purpose, and causes learning of the estimation model, so that it is possible to appropriately measure the body shape size of the user for each purpose.

The data acquired by the acquisition unit 131 is data in which information indicating the length of the line segment estimated from the three-dimensional model and information indicating the actual measurement value of the length of the line segment of the measurement target are collected for each user.

As a result, the information processing device 100 according to the embodiment can appropriately improve the accuracy of the estimation model for estimating the body shape size of the user.

The three-dimensional model is a model in which the entire size is estimated by measuring a part of the size.

As a result, the information processing device 100 according to the embodiment can appropriately generate the three-dimensional model.

The user is a user who provides the length of the line segment of the measurement target estimated from the three-dimensional model and the actual measurement value of the length of the line segment of the measurement target.

As a result, the information processing device 100 according to the embodiment can appropriately improve the accuracy of the estimation model for estimating the body shape size of the user on the basis of the data collected from the user.

The learning unit 133 causes learning of, as training data, the length of the line segment of the measurement target estimated from the three-dimensional models of the plurality of users and the actual measurement value of the length of the line segment of the measurement target.

As a result, the information processing device 100 according to the embodiment can appropriately improve the accuracy of the estimation model for estimating the body shape size of the user by causing learning as training data.

The acquisition unit 131 acquires information indicating a range determined by designating a range being the measurement target by the business operator on the user interface on which the three-dimensional model is displayed.

As a result, the information processing device 100 according to the embodiment can designate the range being the measurement target on the user interface, so that improvement of usability can be promoted.

The information processing device 100 according to the embodiment further includes the estimation unit 134 that estimates the actual measurement value of the length of the line segment of the measurement target of the target user who is the user by inputting the length of the line segment of the measurement target estimated from the three-dimensional model of the target user to the estimation model learned by the learning unit 133.

As a result, the information processing device 100 according to the embodiment can appropriately estimate the length of the line segment of the measurement target of the target user using the learned estimation model.

The information processing device 100 according to the embodiment further includes the estimation unit 134 that estimates the actual measurement value of the length of the line segment of the measurement target of the target user who is different from the user by inputting the length of the line segment of the measurement target estimated from the three-dimensional model of the target user to the estimation model learned by the learning unit 133.

As a result, the information processing device 100 according to the embodiment can appropriately estimate the length of the line segment of the measurement target of the target user using the learned estimation model.

The user is a user having edema in the measurement target due to a predetermined symptom. The target user is a user having edema in the measurement target due to a predetermined symptom.

As a result, the information processing device 100 according to the embodiment can appropriately estimate the length of the line segment of the measurement target of the target user even when the target user has edema and there is swelling in the measurement target range.

### [10. Effect (2)]

As described above, the information processing device 100 according to the embodiment includes the acquisition unit 131 and the learning unit 133. The acquisition unit 131 may acquire information indicating a range of a length measurement target from the three-dimensional model of the user and data of the length of a line segment of the measurement target from the business operator. The learning unit 133 may cause learning of the estimation model that estimates the length of the line segment of the measurement target from the three-dimensional model such that the length of the line segment estimated from the three-dimensional model becomes an actual measurement value of the length of the line segment of the measurement target.

As a result, the information processing device 100 according to the embodiment acquires the information indicating the range being the measurement target and the data of the length of the line segment of the measurement target from the business operator and causes learning of the estimation model, so that it is possible to appropriately measure the body shape size of the user for each business operator.

The data acquired by the acquisition unit 131 may be data in which information indicating the length of the line segment estimated from the three-dimensional model and information indicating the actual measurement value of the length of the line segment of the measurement target are collected for each user.

As a result, the information processing device 100 according to the embodiment can appropriately improve the accuracy of the estimation model for estimating the body shape size of the user.

The three-dimensional model may be a model in which the entire size is estimated by measuring a part of the size.

As a result, the information processing device 100 according to the embodiment can appropriately generate the three-dimensional model.

The user may be a cooperator who provides the length of the line segment of the measurement target estimated from the three-dimensional model and the actual measurement value of the length of the line segment of the measurement target in order to cause learning of the estimation model.

As a result, the information processing device 100 according to the embodiment can appropriately improve the accuracy of the estimation model for estimating the body shape size of the user on the basis of the data of the user who is a data collection cooperator.

The learning unit 133 may cause learning of, as training data, the length of the line segment of the measurement target estimated from the three-dimensional models of the plurality of users and the actual measurement value of the length of the line segment of the measurement target.

As a result, the information processing device 100 according to the embodiment can appropriately improve the accuracy of the estimation model for estimating the body shape size of the user by causing learning as training data.

The acquisition unit 131 may acquire information indicating a range determined by designating a range being the measurement target by the business operator on the user interface on which the three-dimensional model is displayed.

As a result, the information processing device 100 according to the embodiment can designate the range being the measurement target on the user interface, so that improvement of usability can be promoted.

The information processing device 100 according to the embodiment may further include the estimation unit 134 that estimates the actual measurement value of the length of the line segment of the measurement target of the target user who is different from the user by inputting the length of the line segment of the measurement target estimated from the three-dimensional model of the target user to the estimation model learned by the learning unit 133.

As a result, the information processing device 100 according to the embodiment can appropriately estimate the length of the line segment of the measurement target of the target user using the learned estimation model.

The user is a user having edema in the measurement target due to a predetermined symptom. The target user may be a user having edema in the measurement target due to a predetermined symptom.

As a result, the information processing device 100 according to the embodiment can appropriately estimate the length of the line segment of the measurement target of the target user even when the target user has edema and there is swelling in the measurement target range.

### [11. Hardware configuration]

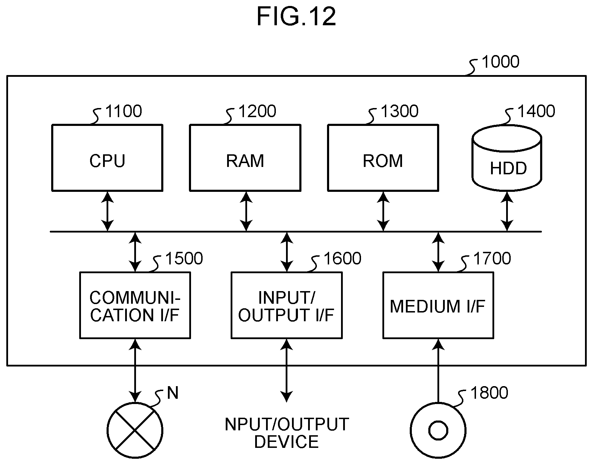

The business operator terminal 10, the first user terminal 20, the second user terminal 30, and the information processing device 100 according to the above-described embodiment are implemented by, for example, a computer 1000 having a configuration as illustrated in FIG. 12. FIG. 12 is a hardware configuration diagram illustrating an example of a computer that implements the functions of the business operator terminal 10, the first user terminal 20, the second user terminal 30, and the information processing device 100. The computer 1000 includes a CPU 1100, a RAM 1200, a ROM 1300, an HDD 1400, a communication interface (I/F) 1500, an input/output interface (I/F) 1600, and a medium interface (I/F) 1700.

The CPU 1100 operates on the basis of a program stored in the ROM 1300 or the HDD 1400, and controls each unit. The ROM 1300 stores a boot program executed by the CPU 1100 when the computer 1000 is activated, a program depending on hardware of the computer 1000, and the like.

The HDD 1400 stores a program executed by the CPU 1100, data used by the program, and the like. The communication interface 1500 acquires data from another device via a predetermined communication network, transmits the data to the CPU 1100, and transmits the data generated by the CPU 1100 to another device via a predetermined communication network.

The CPU 1100 controls an output device such as a display or a printer and an input device such as a keyboard or a mouse via the input/output interface 1600. The CPU 1100 acquires data from the input device via the input/output interface 1600. The CPU 1100 outputs the generated data to the output device via the input/output interface 1600.

The medium interface 1700 reads a program or data stored in the recording medium 1800 and provides the program or data to the CPU 1100 via the RAM 1200. The CPU 1100 loads the program from the recording medium 1800 onto the RAM 1200 via the medium interface 1700, and executes the loaded program. The recording medium 1800 is, for example, an optical recording medium such as a digital versatile disc (DVD) or a phase change rewritable disk (PD), a magneto-optical recording medium such as a magneto-optical disk (MO), a tape medium, a magnetic recording medium, a semiconductor memory, or the like.

For example, in a case where the computer 1000 functions as the business operator terminal 10, the first user terminal 20, the second user terminal 30, and the information processing device 100 according to the embodiment, the CPU 1100 of the computer 1000 executes the functions of the control units 14, 24, 34, and 130 by executing a program loaded on the RAM 1200. The CPU 1100 of the computer 1000 reads and executes these programs from the recording medium 1800, but as another example, these programs may be acquired from another device via a predetermined communication network.

### [12. Others]

Among the processes described in the above embodiments, all or a part of the processes described as being automatically performed can be manually performed, or all or a part of the processes described as being manually performed can be automatically performed by a known method. The processing procedure, specific name, and information including various data and parameters illustrated in the document and the drawings can be changed as desired unless otherwise specified. For example, the various types of information illustrated in each drawing are not limited to the illustrated information.

Each component of each device illustrated in the drawings is functionally conceptual, and is not necessarily physically configured as illustrated in the drawings. That is, a specific form of distribution and integration of each device is not limited to the illustrated form, and all or a part thereof can be functionally or physically distributed and integrated in any unit according to various loads, usage conditions, and the like.

The above-described embodiments can be appropriately combined within a range in which the processing contents do not contradict each other.

Although some of the embodiments of the present application have been described in detail with reference to the drawings, these are merely examples, and the present invention can be implemented in other forms subjected to various modifications and improvements based on the knowledge of those skilled in the art, including the aspects described in the disclosure of the invention.

The "Part (section, module, unit)" described above can be read as "means", "circuit", or the like. For example, the acquisition unit can be replaced with acquisition means or an acquisition circuit.

Note that the following configurations also belong to the technical scope of the present disclosure.

In addition, the following configurations also belong to the technical scope of the present disclosure.
(1) An information processing device including:
   an acquisition unit that acquires information indicating a range being a length measurement target from a three-dimensional model of a user and data of a length of a line segment of the measurement target from a business operator; and
   a learning unit that causes learning of an estimation model that estimates a length of the line segment of the measurement target from the three-dimensional model such that the length of the line segment estimated from the three-dimensional model becomes an actual measurement value of the length of the line segment of the measurement target.
(2) The information processing device according to (1) described above, in which
   data acquired by the acquisition unit is data in which information indicating the length of the line segment estimated from the three-dimensional model and information indicating the actual measurement value of the length of the line segment of the measurement target are collected for each user.
(3) The information processing device according to (1) or (2) described above, in which
   the three-dimensional model is a model in which the entire size is estimated by measuring a part of a size.
(4) The information processing device according to any one of (1) to (3) described above, in which
   the user is a cooperator who provides the length of the line segment of the measurement target estimated from the three-dimensional model and the actual measurement value of the length of the line segment of the measurement target in order to cause learning of the estimation model.
(5) The information processing device according to (4) described above, in which
   the learning unit
   causes learning of, as training data, the length of the line segment of the measurement target estimated from the three-dimensional model of a plurality of the user and the actual measurement value of the length of the line segment of the measurement target.
(6) The information processing device according to any one of (1) to (5) described above, in which
   the acquisition unit
   acquires information indicating a range determined by designating a range being the measurement target by the business operator on a user interface on which a three-dimensional model is displayed.
(7) The information processing device according to any one of (1) to (6) described above, further including
   the estimation unit that estimates the actual measurement value of the length of the line segment of the measurement target of the target user who is different from the user by inputting the length of the line segment of the measurement target estimated from the three-dimensional model of the target user to the estimation model learned by the learning unit.
(8) The information processing device according to (7) described above, in which
   the user is a user having edema in the measurement target due to a predetermined symptom, and
   the target user is a user having edema in the measurement target due to the predetermined symptom.
(9) An information processing method executed by a computer including:
   an acquisition step of acquiring information indicating a range being a length measurement target from a three-dimensional model of a user and data of a length of a line segment of the measurement target from a business operator; and
   a learning step of causing learning of an estimation model that estimates a length of the line segment of the measurement target from the three-dimensional model such that the length of the line segment estimated from the three-dimensional model becomes an actual measurement value of the length of the line segment of the measurement target.
(10) An information processing program that causes a computer to execute:
   an acquisition procedure of acquiring information indicating a range being a length measurement target from a three-dimensional model of a user and data of a length of a line segment of the measurement target from a business operator; and
   a learning procedure of causing learning of an estimation model that estimates a length of the line segment of the measurement target from the three-dimensional model such that the length of the line segment estimated from the three-dimensional model becomes an actual measurement value of the length of the line segment of the measurement target.

### Reference Signs List

- 1: INFORMATION PROCESSING SYSTEM
- 10: BUSINESS OPERATOR TERMINAL
- 11: COMMUNICATION UNIT
- 12: INPUT UNIT
- 13: OUTPUT UNIT
- 14: CONTROL UNIT
- 20: FIRST USER TERMINAL
- 21: COMMUNICATION UNIT
- 22: INPUT UNIT
- 23: OUTPUT UNIT
- 24: CONTROL UNIT
- 30: SECOND USER TERMINAL
- 31: COMMUNICATION UNIT
- 32: INPUT UNIT
- 33: OUTPUT UNIT
- 34: CONTROL UNIT
- 100: INFORMATION PROCESSING DEVICE
- 110: COMMUNICATION UNIT
- 120: STORAGE UNIT
- 121: MODEL LEARNING DATA STORAGE UNIT
- 122: SECOND USER INFORMATION STORAGE UNIT
- 130: CONTROL UNIT
- 131: ACQUISITION UNIT
- 132: GENERATION UNIT
- 133: LEARNING UNIT
- 134: ESTIMATION UNIT
- 135: PROVISION UNIT
- 141: RECEPTION UNIT
- 142: TRANSMISSION UNIT
- 241: RECEPTION UNIT
- 242: TRANSMISSION UNIT
- 341: RECEPTION UNIT
- 342: TRANSMISSION UNIT
- N: NETWORK

## Claims

1. An information processing device comprising:
an acquisition unit that acquires information indicating a range being a length measurement target from a three-dimensional model of a user and data of a length of a line segment of the measurement target, the data being of a length according to a purpose and used for a predetermined purpose; and
a learning unit that causes learning of an estimation model that estimates a length of the line segment of the measurement target used for the purpose from the three-dimensional model such that the length of the line segment estimated from the three-dimensional model becomes an actual measurement value of the length of the line segment of the measurement target.

2. The information processing device according to claim 1, wherein
data acquired by the acquisition unit is data in which information indicating the length of the line segment estimated from the three-dimensional model and information indicating the actual measurement value of the length of the line segment of the measurement target are collected for each user.

3. The information processing device according to claim 1, wherein
the three-dimensional model is a model in which the entire size is estimated by measuring a part of a size.

4. The information processing device according to claim 1, wherein
the user is a user who provides the length of the line segment of the measurement target estimated from the three-dimensional model and the actual measurement value of the length of the line segment of the measurement target.

5. The information processing device according to claim 4, wherein
the learning unit
causes learning of, as training data, the length of the line segment of the measurement target estimated from the three-dimensional model of a plurality of the user and the actual measurement value of the length of the line segment of the measurement target.

6. The information processing device according to claim 1, wherein
the acquisition unit
acquires information indicating the range determined by designating a range being the measurement target by a business operator on a user interface on which the three-dimensional model is displayed.

7. The information processing device according to claim 1, further comprising
an estimation unit that estimates the actual measurement value of the length of the line segment of the measurement target of a target user who is the user by inputting the length of the line segment of the measurement target estimated from the three-dimensional model of the target user to the estimation model learned by the learning unit.

8. The information processing device according to claim 1, further comprising
an estimation unit that estimates the actual measurement value of the length of the line segment of the measurement target of a target user who is different from the user by inputting the length of the line segment of the measurement target estimated from the three-dimensional model of the target user to the estimation model learned by the learning unit.

9. The information processing device according to claim 1, wherein
the user is a user having edema in the measurement target due to a predetermined symptom, and
the target user is a user having edema in the measurement target due to the predetermined symptom.

10. An information processing method executed by a computer comprising:
an acquisition step of acquiring information indicating a range being a length measurement target from a three-dimensional model of a user and data of a length of a line segment of the measurement target, the data being of a length according to a purpose and used for a predetermined purpose; and
a learning step of causing learning of an estimation model that estimates a length of the line segment of the measurement target used for the purpose from the three-dimensional model such that the length of the line segment estimated from the three-dimensional model becomes an actual measurement value of the length of the line segment of the measurement target.

11. An information processing program that causes a computer to execute:
an acquisition procedure of acquiring information indicating a range being a length measurement target from a three-dimensional model of a user and data of a length of a line segment of the measurement target, the data being of a length according to a purpose and used for a predetermined purpose; and
a learning procedure of causing learning of an estimation model that estimates a length of the line segment of the measurement target used for the purpose from the three-dimensional model such that the length of the line segment estimated from the three-dimensional model becomes an actual measurement value of the length of the line segment of the measurement target.
